# EUROPEAN PATENT SPECIFICATION

(11) **EP 0 173 215 B1**
(45) Date of publication and mention of the grant of the patent: **09.06.1993**
(21) Application number: 85110371.3
(22) Date of filing: 19.08.1985
(51) Int. Cl.: C12P 21/02, C12N 15/18

(54) **Method for recovering purified growth hormones from genetically engineered microorganisms**
Verfahren zur Rückgewinnung von gereinigten Wachstumshormonen aus genetisch modifizierten Mikro-organismen
Méthode de récupération d'hormones de croissance purifiées à partir de micro-organismes modifiés par génie génétique

(30) Priority: 15.05.1985 US 734450; 27.08.1984 US 644435
(43) Date of publication of application: 05.03.1986
(73) Proprietor: BIO-TECHNOLOGY GENERAL CORPORATION, New York New York 10152 (US)
(72) Inventor: Bartfeld, Daniel, Nes Ziona (IL); Blumberg, Shmaryahu, Rishon Le Zion (IL); Gorecki, Marian, Rehovot (IL); Hadary, Dan, Rishon Lezion (IL); Zeevi, Menachem, Ramat Gan (IL)
(74) Representative: Henkel, Feiler, Hänzel & Partner

(56) References cited:
- EP-A- 0 114 506
- EP-A- 0 131 843
- EP-A- 0 173 280
- ENDOCRINOLOGY, vol. 84, no. 2, February 1969, pages 325-331; U.J. LEWIS et al.: "Aggregate-free human growth hormone. I. Isolation by ultrafiltration"
- J. Chromat. 517 (1990) 57

## Description

One goal of genetic engineering research has been the development of methods for the production in microorganisms of proteins produced naturally only in higher organisms. This goal has been achieved with respect to certain eucaryotic growth hormones or analogs thereof. Strains of genetically modified bacteria, e.g. Escherichia coli, capable of producing upon growth and induction various growth hormones or analogs have been deposited with the American Type Culture Collection (ATCC) in Rockville, MD. Such hormones are of great potential value in agricultural applications, e.g. to increase milk and meat production in farm animals, as well as in medical applications, e.g. to treat humans afflicted by dwarfism.

The production of a growth hormone by a microorganism involves the following general steps: (1) foreign DNA encoded with the genetic instructions for the production of desired hormone is inserted into an appropriate microorganism, such as Escherichia Coli; (2) the microorganism is cultured under suitable conditions and produces and stores the hormone in large quantities; and (3) the hormone is recovered from the microorganism and purified.

Recovery and purification of the hormone generally involves disrupting the cell, separating the growth hormone from other cellular components and further purifying the recovered hormone. Undesired cellular constituents may be digested with enzymes, solubilized with surfactants and separated from the hormone by centrifugation. Finally, the growth hormone can be purified to a varying degree by a variety of techniques which have been developed for the purification of proteins in general. Such techniques include one or more of the following: dialysis, zonal centrifugation, molecular exclusion chromatography, isoelectric precipitation, salting-in and salting-out, solvent fractionation, electrophoretic methods, ion-exchange chromatography and affinity chromatography. These methods vary among themselves both with regard to economic feasibility and the degree of purification achievable.

Pending co-assigned U.S. patent application Serial No. 514,188, filed July 15, 1983, describes a recovery and purification method for bovine growth hormone. That method involves recovering the hormone by mechanically disrupting a blended cell suspension and incubating the precipitate with lysozyme and then with deoxyribonuclease. The hormone is then purified by sonicating the solubilized hormone, clarifying the solution by centrifugation and subjecting the solution containing the hormone to gel chromatography, ion exchange chromatography, dialysis and lyophilization.

The preceding method requires rather large amounts of lysozyme and entails repeated and time-consuming washes in order to yield purified hormone, and even then in only a relatively low yield (about 10-15% based on the weight of the hormone in the cells at the end of fermentation). The preceding method suffers another disadvantage owing to its reliance on deoxyribonuclease digestion. Deoxyribonuclease is an expensive enzyme that has been used to digest cellular DNA during the hormone recovery process. Preparations of deoxyribonuclease have been found to be contaminated with proteases which degrade the desired product during recovery. Use of deoxyribonuclease as in the preceding method may thus reduce the yield of recovered hormone unless additional precautionary steps are taken to inhibit any protease present. Even then, complete inhibition of protease may not be possible. In addition to reducing the product yield, the use of deoxyribonuclease also leads to a product of less than desirable stability, because of protease contamination.

A method for recovering a purified eucaryotic growth hormone or analog thereof has now been developed which is easier to perform, better controlled and less expensive than previous methods. The method of this invention results in the recovery of the desired product in higher yield and in a more stable form than was previously obtainable. The method requires only 2.5%-5% of the amount of lysozyme used in the previous method, thereby facilitating purification and reducing the cost of recovery. Moreover, the method does not require the use of any deoxyribonuclease. In addition to further reducing the cost of recovery, eliminating the need for deoxyribonuclease more than doubles the yield of product and affords the product in significantly more stable form. Furthermore, by reducing the number of washings required this method affords a more convenient and time-efficient process for recovery of purified growth hormones or analogs, taking only 10% to 20% of the time of the previous method. By utilizing ultrafiltration, the method achieves both a significant reduction in cost and improved product purity.

EP-A-0 114 506 discloses a means for enhancing the amount of protein precipitated in refractile bodies, and further discloses a method of isolating active enzyme from the refractile bodies by solubilization in a strong chemical denaturant followed by further purification and renaturation steps.

### Summary of the Invention

This invention concerns a method for recovering a purified animal or human growth hormone or a polypeptide analog thereof from a bacterial cell in which the eucaryotic growth hormone or analog has been produced by means of expression of a DNA sequence encoding the hormone or analog which comprises the following steps:
a) disrupting the cell wall of the bacterial cell or fragments thereof to produce a lysate;
b) separating a precipitate containing particulate and insoluble material from the lysate;
c) preparing a suspension of the separated precipitate in an appropriate buffer solution;
d) contacting the suspension for a suitable period of time with up to 100 µg lysozyme/ml solution in the absence of DNAse and treating the suspension to effect contact between liquid and solids;
e) separating partially or completely precipitated material from the liquid solids contacted suspension;
f) solubilizing the separated precipitate in a sufficient amount of aqueous solution and adjusting the pH to between 9.0 and 12.0 to form a solution;
g) separating the solubilized hormone or analog from other cellular components by ultrafiltration using a membrane having a cut-off point of about 100 K molecular weight under suitable conditions to produce an ultrafiltrate containing the hormone or analog and a retentate; and
h) treating the solubilized hormone or analog so as to further purify and concentrate the hormone or analog and thereby recover the purified hormone or analog.

Lysozyme may be employed in step (d) to treat the suspension of the precipitate separated from the lysate before liquid-solids contact and subsequent precipitate separation. The latter two steps as well as ultrafiltration may be repeated. Ion exchange chromatography may be employed in step (h) to further purify and concentrate the hormone or analog. Additionally, most of the process steps may be performed in a continuous flow and may be automated.

Suitable hormone-producing bacterial cells for use in this invention include Escherichia coli strains ATCC Nos. 39806, 39785, 39386, 39384, 39804, and 39792. Growth hormones which can be recovered and purified using this invention include bovine, porcine, chicken, and human growth hormones, and analogs thereof.

### Detailed Description of the Invention

A new method has been developed for recovering a purified eucaryotic, e.g. animal or human growth hormone or an analog thereof from a bacterial cell in which the growth hormone or analog has been produced by means or expression of a DNA sequence encoding the hormone or analog.

Genetically modified bacterial cells for use in this method are known. A strain of Escherichia coli genetically modified to produce an analog of bovine growth hormone (bGH), for example, has been deposited with the American Type Culture Collection (ATCC, Rockville, MD) and has been assigned ATCC No. 39806. The bGH analog has the amino acid sequence Met-Asp-Gln added to the N-terminus of the phenylalanine form of natural bGH. Another genetically modified E. coli cell line deposited with ATCC and assigned ATCC No. 39785 produces a bGH analog having a methionine residue added to the N-terminus of the phenylanine form of natural bGH. Still another strain of E. coli deposited with ATCC and assigned ATCC No. 39386 produces a human growth hormone (hGH) analog having the amino acid sequence of natural hGH beginning with Met¹⁴ and lacking amino acids 1-13, while the E. coli cell line assigned ATCC No. 39384 produces a hGH analog having the sequence of natural hGH preceded by methionine at the N-terminus. Similarly, the E. coli cell lines assigned ATCC Nos. 39804 and 39792 produce a porcine growth hormone (pGH) analog and a chicken growth hormone (cGH) analog respectively, each having a methionine residue added to the N-terminus of the phenylanine form of the respective natural hormone. Each of the aforementioned hormone analogs may be produced upon growth and induction of the appropriate cells under suitable conditions and may be recovered in purified form by the method of this invention.

This method is easier to perform, better controlled and less expensive than previous methods and results in the recovery of the desired product in higher yield and in a more stable form. Such products may be recovered by this method in yields in excess of 25% or even 30% by weight based on the weight of the hormone or analog present in the bacteria at the end of fermentation. Lysozyme is utilized in lesser amounts of the enzyme than required in previous methods. Also the method does not require the use of any deoxyribonuclease, may be performed in a continuous flow manner and may be conveniently automated.

In accordance with this method, after the growth, induction and harvest of appropriate bacterial cells the decanted bacterial cells, fragments thereof or cell cake are suspended in a suitable buffered solution at a neutral pH, e.g. a pH of about 7.4. A suitable suspension medium for this purpose is an aqueous solution of 50mM Tris (pH 7.4):50 mM NaCl, 50mM Tris (pH 7.4) : 50mM EDTA, 50mM sodium phosphate buffer (pH 7.4):50mM NaCl or 50mM sodium phosphate buffer (pH 7.4) : 50mM EDTA. Alternatively, the fermentation mash cell suspension, after adjusting its pH to a neutral pH, may be used directly.

The cell wall of the suspended bacterial cell or fragments thereof is then disrupted to produce a lysate. In a preferred embodiment, the cells are mechanically disrupted at temperatures less than about 35^{o}C, e.g. between about -10^{o}C and about +8^{o}C. A bead mill or homogenizer, e.g. a Dynomill KD-5 apparatus (Willy A. Bachofen, Basel) is an appropriate, commercial disruptor for this purpose. The disruption is preferably performed by continuously passing the suspension through the disruptor at an appropriate flow rate, e.g.about 80 L/hr.

A precipitate containing particulate and insoluble material including the hormone or analog and other water insoluble proteins as well as cell membrane and cell wall fragments and aggregates is then separated from the lysate which also contains water soluble proteins, DNA and RNA or fragments thereof. To effect this separation, the lysate is preferably diluted with a suitable amount, e.g. an approximately equal volume, of deionized water and then centrifuged in a solid bowl continuous flow centrifuge (e.g. Cepa 101) at flow rates sufficient to effect the desired separation, e.g. about 60-80 L/hr. A suspension is then prepared of the separated precipitate in a suitable amount of an appropriate buffer solution having a neutral pH, e.g. a pH of about 7.4. Preferably the solution contains 50 mM Tris or sodium phosphate (pH 7.4):50 mM EDTA. A suitable amount of buffer solution for the suspension is about 0.5-2.5 liters per Kg of initial bacterial cells, preferably about 1.2 liters per Kg of cells.

Undesirable cellular material such as cell wall fragments may then be digested by incubating the suspension with an appropriate enzyme capable of digesting polysaccharides present in the suspension, e.g. lysozyme (Sigma L-6876, St. Louis, MO), under suitable conditions and for a suitable period of time. The presently preferred concentration of lysozyme is about 50-100µg per ml of solution, a significant reduction from the 2 mg/ml used in previous methods. An appropriate period of time for the lysozyme incubation is between about one and 20 hours. The temperature during this incubation period is preferably maintained at about 37^{o}C. A suitable amount of an appropriate surfactant, e.g. Triton® X-100 (Rohm & Haas Co., Philadelphia, PA), may then be added to the suspension to a final concentration of about 1% (v/v) in order to solubilize material such as cellular lipids. The suspension is then further incubated for a suitable period of time, e.g. about 30 minutes.

The liquid and solids contained in the suspension are then treated so as to effect intimate liquid-solids contact, for example, by sonication under suitable conditions, such as with a 370W continuous flow sonicator (Heat Systems Ultrasonics, Plainview, NY) set at 65% of maximum power and with a flow rate of about 18 L/hr to 25 L/hr. Precipitated material is then separated from the liquid-solids contacted suspension, preferably by centrifuging the liquid-solids contacted suspension in a solid bowl continuous flow centrifuge (e.g. Cepa 101) at an appropriate flow rate e.g. about 30 L/hr. In another embodiment, the separation in this and subsequent steps may be effected by microfiltration with an appropriate membrane, e.g. a Millipore Durapore 0.45 micron membrane instead of centrifugation. After the separation, the precipitated material separated from the liquid-solids contacted, e.g. sonicated, suspension may be washed, suitably with 50 mM Tris or phosphate (pH 7.4):100 mM NaCl, and resuspended in a suitable amount of an appropriate medium, e.g. a buffered or unbuffered aqueous solution. The suspension so prepared is then brought into liquid-solids contact, such as through sonication, under suitable conditions, and the resultant precipitated material is partially or completely separated from the liquid-solids contacted, e.g. sonicated, suspension, e.g. by centrifugation or ultrafiltration as above. The re-suspending liquid-solids contacting (e.g. sonicating) and separating are preferably carried out at least one additional time. In an especially preferred embodiment these steps are performed four times as follows: the resultant precipitate is first re-suspended in 20 mM Tris (pH 7.4) or 50mM sodium phosphate buffer (pH 7.4) and the suspension liquid-solids contacted (e.g. sonicated) as above. The suspension is then centrifuged as above and the recovered precipitate re-suspended in 50 mM Tris (pH 7.4) :100 mM NaCl or 20 mM sodium phosphate buffer (pH 7.4):10 mM EDTA:100 mM NaCl. This suspension is then liquid-solids contacted, such as by sonication, and centrifuged as above and the recovered precipitate resuspended in deionized water. The water suspension is liquid-solids contacted, e.g. sonicated and centrifuged, again as above, and the recovered precipitate is again suspended in deionized water. This second water suspension is then liquid-solids contacted, e.g. sonicated and centrifuged as above to yield a precipitate enriched in the desired hormone or analog.

The separated precipitate is then solubilized, for example, by resuspending the precipitate, at an appropriate temperature, e.g. 4-40^{o}C, in a sufficient amount of water, preferably about twenty-five (25) volumes, and the pH adjusted to a suitable alkaline pH, e.g. about 9.0 to 12.0 to form a solution containing the hormone or analog. High sheer agitation (e.g. a Polytron [Kinematica] blender) may be used to facilitate the dissolution. The pH of the initial suspension may be suitably adjusted by adding NaOH to the suspension, e.g. 1-10 N NaOH, until the pH is about 9.0-12.0, preferably about 11.8-12.0. Preferably the NaOH is added simultaneously to the solution with vigorous stirring. The solution is then incubated and stirred for about 1 hour, after which the pH may be adjusted to about 10.5.

An appropriate amount of a suitably concentrated aqueous solution of sodium borate, e.g. about 1M (an appropriate pH, e.g. 11.5-12.0), is then added to the solution to achieve a final borate concentration preferably of about 10 mM. The protein solution is then titrated to pH 10.5 either by its dilution with 2-10 volumes of 10 mM sodium borate buffer (pH 10.2) or by titration with concentrated HCl or 1N HCl. The solution is then further incubated for 1-12 hours with stirring. The solution so obtained may be clarified if desired by sonicating the solution under appropriate conditions, e.g. with a continuous flow Heat System Sonicator Model 370 at a flow rate of about 10 L/hr; centrifuging the sonicated solution under suitable conditions, e.g. with a solid bowl continuous flow centrifuge (e.g. Cepa 101) at a flow rate of about 30 L/hr; separating the supernatant liquid and vacuum filtering the liquid, e.g. through a Whatman No. 2 paper on a Buchner vacuum filter to produce a clarified solution containing the solubilized hormone or analog. Alternatively, centrifuging may be employed exclusively.

The solubilized hormone or analog is then separated from other cellular components such as proteins, lipids and other particles, and aggregates are dissociated by ultrafiltration under suitable conditions. Preferably, the retentate obtained is resubjected to ultrafiltration at least once. During this procedure, the volume of the retentate should be reduced by about 95%, e.g., from about 10 liters to about 0.5 liters and the filtrate collected. A Pellicon® Cassette or PUF-100 spiral (Millipore Corp., Bedford, MA) with a 100K M.W. cut-off is suitable for use in the ultrafiltration procedure. Preferably the retentate is further diluted with a suitable buffer at an appropriate alkaline pH, e.g. about 9-12, and subjected to further ultrafiltration. In a presently preferred embodiment, 10 mM borate buffer (pH 10.5-12.0) is used. Redilution and reconcentration by ultrafiltration is preferably repeated at least once. Alternately, the ultrafiltration may be effected one or more times at substantially constant retentate volume. In an especially preferred embodiment, ultrafiltration is repeated until the absorbance of the retentate at 280 nanometers is less than about 0.1 optical density units. The hormone may be conveniently obtained from the ultrafiltrate at a level of purity and biological activity superior to that obtained previously by gel chromatography.

The purified hormone or analog obtained in the ultrafiltrate is then treated, such as by ion exchange chromatography, e.g. with an amine-containing ion exchange resin such as diethylaminoethyl, so as to further purify and concentrate the hormone or analog and recover the purified hormone or analog. Concentration of the ultrafiltration using a 10K M.W. membrane to remove water is optional prior to ion exchange chromatography. During the ion exchange chromatography step remaining DNA, lysozyme if present and multimers are separated from the hormone or analog. In this step the pH of the ultrafiltrate is first adjusted to a suitable pH, e.g. pH 9.0, by titrating with hydrochloric acid, and is loaded on a suitable ion exchange column, e.g. a DEAE-Sepharose® CL-6B (fast flow) KS-370 sectional column (Pharmacia Fine Chemicals, Piscataway, NJ). The system is then washed with a suitable buffer, e.g. 10 mM borate buffer, and the sample eluted at a suitable rate with an appropriate eluent, e.g. 10 mM borate buffer (pH 9.0) containing 100 mM NaCl. The fractions containing the hormone or analog may be concentrated by ultrafiltration using a Pellicon® Cassette or PUF-100 spiral with a 10K M.W. cut-off and may then be dialyzed against a solution containing a suitable concentration of an appropriate salt, e.g. 3 mM ammonium hydroxide or ammonium bicarbonate, again using a 10K M.W. cut-off Pellicon® Cassette. The dialyzed material may then be dried by lyophilization or by spray drying under appropriate conditions. Spray drying is the more economical process and may be conveniently accomplished in a minor spray dryer (Niro Atomyzer) using an inlet temperature of about 200^{o}C, an outlet temperature of about 50^{o}C and a flow rate of about 2.5 L/hr. Animal growth hormones or analogs so processed may thus be obtained in the form of a white fine powder.

Purified animal growth hormones or analogs may be recovered by this method in yields greater than or equal to about 30% based on the weight of the hormone or analog present in the bacteria at the end of fermentation. The hormone or analog so obtained appears as one spot when developed on 15% polyacrylamide-SDS gel against low molecular weight size markers (Pharmacia) as a reference.

The peptides obtained after ultrafiltration and ion exchange chromatography according to this invention were purer and more stable than when gel chromatography was used in place of ultrafiltration or when concentration of the peptides was performed with a 10K M.W. ultrafiltration in place of ion exchange chromatography.

With regard to biological activity of the hormones and analogs recovered, radioimmunoassay results indicate that antibodies to the natural hormone had approximately equal affinities to the hormone or analog produced by the appropriate microorganism and recovered in accordance with this invention. Similarly, radioreceptor binding assays indicate binding activity of appropriate membranes toward the hormones or analogs purified by the present invention equivalent to that toward authentic samples of the natural hormones. Moreover, no antibodies to the hormones or analogs were detected following either primary or booster injections of the peptides into appropriate animals. Furthermore, the bovine growth hormone analog was found to increase lactation when administered to lactating cows.

The examples which follow are set forth to aid in understanding the invention but are not intended to, and should not be construed as to, limit its scope in any way. The examples do not include detailed descriptions for conventional methods which are well-known to those skilled in the art.

### EXAMPLE 1

Growth of E. coli and Production of a bGH Analog

### I. Stock Cultures

Stock cultures of E. coli strain ATCC No. 39806 which upon growth and induction produces the bGH analog, Met-Asp-Gln-bGH were grown on casein medium (see Inoculum), then diluted two-fold with freezing medium and stored at -80^{o}C. Freezing medium contains per 500 ml:

| | |
|---|---|
| K₂HPO₄ | 6.3 gr |
| KH₂PO₄ | 1.8 gr |
| Na Citrate | 0.45 gr |
| MgSO₄·7H₂O | 0.09 gr |
| (NH₄)₂SO₄ | 0.9 gr |
| Glycerol | 44.0 gr |

### II. Inoculum

The inoculum was propagated in 20 g/l casein hydrolysate, 10 g/l yeast extract and 2 gl NaCl. Sterile medium in shaker flask was inoculated from stock culture and incubated 15 hours on a shaker at 30^{o}C at approximately 200 r.p.m. As needed subsequent stages in inoculum propagation were carried out in stirred aerated fermenters. Sterile medium was inoculated with 2-10% inoculum culture, and incubated 15 hours at 30^{o}C, pH 7 ± 0.5 with agitation and aeration to maintain a dissolved oxygen level about 20% air saturation.

### III. Production

The production medium contains:

| | |
|---|---|
| Case in hydrolysate | 20 g/l |
| Yeast extract | 10 g/l |
| K₂HPO₄ | 2.5 g/l |
| MgSO₄·7H₂O | 1 g/l |
| NaCl | 5 g/l |
| Biotin | 0.1 mg/l |
| Thiamine | 1 mg/l |
| Trace elements solution | 3 ml/l |

The medium also contains 100 mg/liter ampicillin. The ampicillin is optional for production but is always found in the medium used for growing the inoculum.

Biotin, thiamine, and ampicillin in concentrated solutions were filter sterilized separately and added to the sterile production medium before inoculation. Sterile glucose solution was added initially to supply 10 g/l. At the induction step another 10 g/l of glucose was added.

The trace elements solution contains:

| | |
|---|---|
| FeCl₃ | 16 g/l |
| ZnCl₂·4H₂O | 2 g/l |
| CoCl₂·6H₂O | 2 g/l |
| Na₂MoO₄·2H₂O | 2 g/l |
| CaCl₂·2H₂O | 1 g/l |
| CuCl₂ | 1 g/l |
| H₃BO₃ | 0.5 g/l |
| Conc. HCl | 100 ml/l |

The medium is inoculated with 0.5-10% inoculum culture and incubated at 30^{o}C. Agitation-aeration rates are set to maintain a dissolved oxygen level above 20% air saturation. The pH is maintained at 7.0 to 7.5 ± 0.2 with NH₃. Once cell concentration reaches about 3.5 g/l (OD₆₆₀ = 10) induction is started by raising the temperature to 42^{o}C. The temperature was then maintained at 42^{o}C for 1-5 hours, after which time the cells may be harvested from the cell suspension.

### EXAMPLE 2

### Cell Harvesting

Five hundred liters of cell suspension (containing 6.15 DCW/L and 490 mg bGH/L) obtained as in Example 1 was cooled to room temperature and transferred to a holding tank. The cell suspension was centrifuged at 14,000 RPM (16,000 xg) on a CEPA 101 tubular bowl centrifuge at a feed rate of 250 liters/hr. The CEPA 101 centrifuge had a tube length of 73.7 cm, a radius to the discharge dam of 3.25 cm and a radius to the bowl wall of 7.35 cm. It was equipped with acceleration fins and had cooling on the exterior jacket.

The clear supernatant had a density of 1.0 g/ml, contained no detectable bGH and was discarded. The cake, weighing 11.4 kg (75% wet), contains the aggregated bGH and was saved. The cake was typically about 2 cm in thickness with a density of 1.1 g/ml. An alternative procedure is to allow the cells to settle overnight and siphon off the clear supernatant.
Assay methods: Quantitated gel scan on cell suspension, supernatant and cake.
Step yield¹: 100% Overall yield: 100%
Output volume: 2.3% of initial cell suspension volume
Calculated assay: 21.5 g bGH/kg cake
Enrichment factor: 44 (bGH concentration in output/bGH concentration in input)
¹ Yields were determined by measuring OD units, which are defined as the absorbance of the solution at 280nm (1-cm cell) times the volume of the solution in mL. For example if the OD value is 9.1 and the volume of the solution is 32,600 mL, the OD units value is 297,000 OD. If the absorbance reading is made at pH 9, the OD value may be converted to grams bGH by multiplying with the conversion factor 0.00157, yielding 14.3g bGH/L.

### EXAMPLE 3

### Cell Disruption

The wet cells (11.4 kg) from the cell harvesting step (Example 2) were suspended in 4 liters of 50 mM Tris:50 mM NaCl buffer at pH 7.4 for each kilogram of wet cells (total of 45.6 liters). A Polytron high-shear mixer (Kinematica) aids in dispersing the cake. The suspended cells were fed into Dynomill KD-5 bead mill disrupter (Willy A. Bachofen, Basel) at 80 L/hr. The bead mill was maintained at -10^{o}C to + 8^{o}C by refrigeration. The disrupted cells were then diluted with an equal volume of deionized water before centrifugation on the CEPA 101 tubular bowl centrifuge. The feed rate to the centrifuge was 30-60 L/hr. All centrifugation conditions except feed rate were identical to those of cell harvesting. The supernatant containing about 10% of the input bGH was discarded. The cake, weighing 5.28 kg (75% wet), contains the aggregated bGH and was saved.

### Disruption Buffer (50 mM Tris:50 mM NaCl, pH 7.4):

6.06 g Tris and 2.97 g NaCl in 750 mL deionized water was adjusted to pH 7.4 with concentrated HCl, diluted to 1 L with deionized water, and if necessary readjusted to pH 7.4.
Assay methods: Quantitated gel scan on cells, supernatant and cake
Step yield: 90% Overall yield: 90%
Output volume: 1.05 of initial cell suspension volume
Calculated assay: 37.1 g bGH/kg of cake
Enrichment factor: 1.7

### EXAMPLE 4

### Lysozyme Treatment

The wet cake (5.28 kg) from the disruption step (Example 2) was suspended in 1.2 liters of 50 mM Tris:50 mM EDTA buffer at pH 7.4 for each kilogram of original wet cells harvested (total of 13.7 liters). A Polytron high-shear mixer aids in dispersing the cake. Lysozyme (Sigma Grade II) was added (0.05 g/l of slurry and the slurry mixed at 37^{o}C for one hour. Triton® X-100 (Rohm & Haas Co., Philadelphia, PA) was added (100 ml of a 10% v/v pH 7.4 buffered solution per liter, yielding a final concentration of 1% Triton X-100) and the mixture incubated for an additional 30 minutes. The slurry was sonicated in a Heat Systems 370 Watt sonicator at 65% power equipped with a flow cell. The flow rate through the sonicator was 18 L/hr. The sonicated slurry was centrifuged on the CEPA 101 at 30 L/hr. All centrifugation conditions except feed rate were identical to those of cell harvesting. The supernatant containing about 3% of the input bGH was discarded. The cake, weighing 3.07 kg (75% wet), contains the aggregated bGH and was saved.

### Lysozyme Buffer (50 mM Tris:50 mM EDTA, pH 7.4):

6.06 g Tris and 14.6 g EDTA in 750 mL deionized water was adjusted to pH 7.4 with concentrated HCl or 50% NaOH, diluted to 1 L with deionized water and if necessary, readjusted to pH 7.4.
Assay methods: Quantitated gel scans on supernatants and cakes
Step yield: 97% Overall yield: 87.3%
Output volume: 0.61% of initial cell suspension volume
Calculated assay: 61.9 g bGH/kg of cake
Enrichment factor: 1.7

### EXAMPLE 5

### First Wash

The wet cake (3.07 kg) from the lysozyme step (Example 4) was suspended in 0.6 liters of 20 mM Tris buffer at pH 7.4 for each kilogram of original wet cells harvested (total of 6.8 liters). A Polytron high-shear mixer aids in dispersing the cake. The slurry was sonicated at a flow rate of 18 L/hr through the Heat Systems 370 watt model at 65% power. The sonicated slurry was centrifuged on the CEPA 101 at 30 L/hr. All centrifugation conditions except feed rate were identical to those of cell harvesting. The supernatant was discarded. The cake, weighing, 2.46 kg (75% wet), contains the aggregated bGH and was saved.

### First Wash Buffer (20 mM Tris, pH 7.4):

2.42 g Tris in 750 mL deionized water was adjusted to pH 7.4 with concentrated HCl, diluted to 1 L with deionized water, and readjusted to pH 7.4, if necessary.
Assay methods: Quantitated gel scans on supernatants and cakes
Step yield: 100% Overall yield: 87.3%
Output volume: 0.49 of initial cell suspension volume
Calculated assay: 77.3 g bGH/kg of wet cake
Enrichment factor: 1.2

### EXAMPLE 6

### Salt Wash

The wet cake (2.46 kg) from the first wash step (Example 5) was suspended in 0.6 liters of 50 mM Tris:100 mM NaCl buffer at pH 7.4 for each kilogram of original wet cells harvested (total of 6.8 liters). A Polytron high-shear mixer aids in dispersing the cake. The slurry was sonicated at a flow rate of 18 L/hr through the Heat Systems 370 watt model at 65% power. The sonicated slurry was then centrifuged on the CEPA 101 at 30 L/hr. All centrifugation conditions except feed rate were identical to those of cell harvesting. The supernatant was discarded. The cake, weighing 2.15 kg (75% wet), contains the aggregated bGH and was saved.

### Salt Wash Buffer (50 mM Tris:100 mM NaCl, pH 7.4):

6.06 g Tris and 5.85 sodium chloride in 750 mL deionized water was adjusted to pH 7.4 with concentrated HCl, diluted to 1 L with deionized water, and, if necessary, readjusted to pH 7.4.
Assay methods: Quantitated gel scans on supernatants and cakes
Step yield: 100% Overall yield: 87.3%
Output volume: 0.43% of initial cell suspension volume
Calculated assay: 88.4 g bGH/kg of cake
Enrichment factor: 1.14

### EXAMPLE 7

### First Water Wash

The wet cake (2.15 kg) from the salt wash step (Example 6) was suspended in 1.2 liters of deionized pyrogen-free water for each kilogram of original wet cells harvested (total of 13.7 liters). A Polytron high-shear mixer aids in dispersing the cake. The slurry was sonicated at a flow rate of 18 L/hr through the Heat Systems 370 watt model at 65% power. The slurry pH should increase from the starting water pH of about 6.2 to about pH 7.8-8.0 The sonicated slurry was then centrifuged on the CEPA 101 at a flow rate of 30 L/hr. All centrifugation conditions except feed rate were identical to those of cell harvesting. The supernatant was discarded. The cake, weighing 1.90 kg (75% wet), contains the aggregated bGH and was saved.
Assay methods: Quantitated gel scans on supernatants and cakes
Step yield: 99% Overall yield: 86.4%
Output volume: 0.38% of initial cell suspension volume
Calculated assay: 99.0 g bGH/kg of cake
Enrichment factor: 1.12

### EXAMPLE 8

### Second Water Wash

The wet cake (1.90 kg) from the first water wash step (Example 7) was suspended in 1.2 liters of deionized pyrogen-free water for each kilogram of original wet cells harvested (total of 13.7 liters). A Polytron high-shear mixer aids in dispersing the cake. The slurry pH should be about 8.4. The slurry was sonicated at a flow rate of 18 L/hr through the Heat Systems 370 watt model at 65% power. The sonicated slurry was then centrifuged on the CEPA 101 at a flow rate of 30 L/hr. All centrifugation conditions except feed rate were identical to those of cell harvesting. The supernatant was discarded. The cake, weighing 1.31 kg (75% wet), contains the aggregated bGH and was saved.
Assay methods: Quantitated gel scans on supernatants and cakes
Step yield: 99% Overall yield: 85.6%
Output volume: 0.26% of initial cell suspension volume
Calculated assay: 160 g bGH/kg of cake
Enrichment factor: 1.62

### EXAMPLE 9

### Dissolution Step

The wet cake (1.31 kg) from the second water wash (Example 8) was suspended in 25 liters of deionized pyrogen-free water for each kilogram of washed cake (total of 32.6 liters). A Polytron high-shear mixer aids in dispersing the cake. The pH was adjusted slowly to 11.8 by addition of 10N NaOH. The aggregated bGH dissolved slowly over a period of one hour. After the one hour incubation period, 1M sodium borate at pH 11.8 was added to the solution to make the final solution 10 mM in sodium borate. The solution was held for 30 minutes before sonication through the Heat System 370 watt model at 65% power. The feed rate to the sonicator was 18 L/hr. If the solution was very hazy, it was centrifuged at 30 L/hr through the CEPA 101; otherwise, the solution was filtered in 5-L portions through three 24-cm diameter Whatman No. 2 papers in a single Buchner filter. The vacuum was adjusted to prevent foaming during filtration. There was only a trace of insoluble material. The filtrate (32.6 L) contains the bGH in solution.

The absorbance of the clarified solution was 9.1 O.D. at 280 nm (1-cm cell). If all the absorption were due to bGH, which it is not, this would correspond to 14.3 g bGH/L. The true bGH content is less than half of this value. UV-absorbing impurities account for the difference. An absorbance of 1.0 at 280 nm (1-cm cell) is given by a 1.57 g/l solution of pure bGH at pH 9. (The absorbance of bGH changes with pH).

### Dissolution Buffer (1M sodiuim borate at pH 11.8):

Dissolve 61.8 grams boric acid in 750 ml deionized water and adjust to pH 11.8 with 50% NaOH. Dilute to a final volume of 1 liter with deionized water.
Assay methods: Quantitated gel scan on washed cake and dissolved bGH
Step yield: 100% Overall yield: 85.6%
Output volume: 6.8% of initial cell suspension volume
Calculated assay: 6.2 g bGH/liter
Enrichment factor: 0.039

### EXAMPLE 10

### 100K Ultrafiltration

The clarified protein solution (32.6 liters, 297,000 OD) (Example 9) was divided into separate portions (6 x 5.4 liters) each having an absorbance of 50,000-60,000 OD. Each portion was ultrafiltrated separately through a Pellicon® Cassette System ultrafilter (Millipore, Bedford, MA) equipped with three 100,000 molecular weight cutoff cassettes (type PTHK) of 5 ft² area each. The first portion was concentrated to 1 L retentate volume. The ultrafiltrate was collected, assayed by UV, and saved. The retentate was diluted to its original volume with fresh 10 mM sodium borate buffer at pH 11.8 and mixed well. The batch was concentrated again to 1 L retentate volume. The ultrafiltrate was collected and combined with the first ultrafiltrate. When a running total of the OD absorbance in the ultrafiltrates equaled 20% of the OD absorbance of the initial charge to the ultrafilter, the retentate volume on the next concentration was taken to 0.5 L instead of 1 L. The cycle of concentration and dilution with 10 mM sodium borate buffer was continued until the ultrafiltrate from a retentate volume of 0.5 L had an absorbance at 280 nm (1-cm cell) of less than 0.1. This normally takes between 9 and 12 cycles of ultrafiltration and dilution. The final retentate was discarded. Flow rates for the ultrafiltration are shown in Table 1.

All ultrafiltrates were then combined and adjusted to pH 9.0 with 6N HCl. The other 5.4-L portions were processed through the Pellicon® System in a similar fashion, and all pH-adjusted ultrafiltrates were combined. A typical run produced a total of 380 liters of ultrafiltrate with an absorbance of 0.26 OD/ml, equivalent to a total OD value of 100,000 (pH 12) and required 24 to 40 hours to complete.

**Table 1**

| Flow Rates for 100K M.W. Ultrafiltration¹ | | |
|---|---|---|
| Conditions | Rate per unit of membrane area | Rate with 3 cassettes (15 ft²) |
| Ultrafiltration rate when retentate is most concentrated: | 1.6 L/hr ft² | 24 L/hr |
| Ultrafiltration rate when retentate is most dilute: | 2.4 L/hr ft² | 36 L/hr |
| Recirculation rate when retentate is most concentrated: | 72 L/hr ft² | 18 L/min |
| Recirculation rate when retentate is most dilute: | 36 L/hr ft² | 9 L/min |

| | | |
|---|---|---|
| ¹ At an inlet pressure of 50 psig and a back pressure of 5 psig | | |

Assay methods: Quantitated gel scan on input and absorbance at 280 nm (1-cm cell) on output; qualitative gel chromatography
Step yield: 75.5% Overall yield: 64.6%
Output volume: 76% of initial cell suspension volume
Calculated assay: 0.4 g bGH/liter
Enrichment factor: 0.065
The feed, retentate and ultrafiltrate were each examined by Sepharaose® CL-6B (Pharmacia Fine Chemicals, Piscataway, NJ) gel chromatography in order to determine the extent and quality of the separation.

The conditions of the gel column are:
2.6-cm dia. x 100-cm long
Total volume: 500 mL Void volume: 150 mL
Flow rate: 40 to 60 mL/hr
Loading: 50 OD's in 5 mL
Eluent: 10 mM sodium borate at pH 12
Normal running time: 5 hrs
Detection: 280 nm UV

### Gel chromatography buffer (10 mM sodium borate at pH 12):

Prepared as described in Example 9.

### EXAMPLE 11

### Ion Exchange Chromatography

The combined ultrafiltrates (380 liters, with an absorbance of 100,000 OD) from the 100K ultrafiltration of Example 10 were loaded onto a Sepharose® CL-6B DEAE ion-exchange column (Pharmacia Fine Chemicals, Piscataway, NJ) at a linear flow velocity of 93 cm/hr (100 L/hr). The 37-cm diameter, 15-cm high column was washed with two bed volumes (32 L) of 10 mM sodium borate buffer at pH 9.0, and the eluate from the loading and washing steps discarded. A step change in eluent to 10 mM sodium borate:100mM sodium chloride at pH 9 displaced the bGH from the column. The elution flow velocity was 93 cm/hr. The progress of the elution was monitored by following the absorbance of the eluate at 280 nm. The bGH peak was collected in 4 to 5 bed volumes (84 liters with a total absorbance of 43,000 OD) and saved. The average concentration of the bGH fraction was 0.8 g bGH/L.

The column was regenerated by washing the bed at a velocity of 93 cm/hr as follows: one bed volume of 1M sodium acetate adjusted to pH 3.0 with HCl was applied to the column followed by one and one half bed volumes 0.5 N NaOH. After treatment with the NaOH, the column was allowed to stand for two hours, after which time the column was washed with one and one half bed volumes 1M sodium acetate adjusted to pH 3.0 with HCl. The bed was then equilibrated with 10 mM sodium borate (pH 9) by running at least three bed volumes of buffer through the ion-exchange bed. Conductivity, and pH of the eluate should be checked against the ingoing solution.
Assay methods: Absorbance at 280 nm (1-cm cell)
Step yield: 43% Overall yield: 27.7%
Output volume: 16.8% of initial cell suspension volume
Calculated assay: 0.8 g bGH/L
Enrichment factor: 2

### Column wash buffer (10 mM sodium borate pH 9):

0.62 g boric acid in 750 mL deionized water was adjusted to pH 10 with concentrated HCl, diluted to 1 L with deionized water and, if necessary, readjusted to pH 9.0.

### Elution buffer (10 mM sodium borate, 100mM sodium chloride, pH 9):

3.81 g sodium borate decahydrate and 5.85 g sodium chloride in 750 mL deionized water was adjusted to pH 9.0 with concentrated HCl, diluted to 1 L with deionized water and, if necessary, readjusted to pH 9.0.

### Regeneration buffer: (1M sodium acetate, pH 3.0):

136.09 g sodium acetate ·3H₂O in 750 ml deionized water was adjusted to pH 3.0 with concentrated HCl, diluted to 1 L with deionized water and, if necessary, readjusted to pH 3.0.

### EXAMPLE 12

### Concentration and Dialysis

The bGH fraction from the ion-exchange step (84 liters, 43,000 OD) (Example 11) was adjusted to pH 10.5 with 1N NaOH. The adjusted solution was concentrated by ultrafiltration through a Millipore Pellicon® ultrafilter equipped with three 10,000-molecular weight cutoff cassettes (type PTGC) of 5 ft² each until the calculated absorbance at 280 nm (1-cm cell) of the retentate is 10. The ultrafiltrate should contain no bGH and was discarded. The retentate was usually concentrated by a factor of roughly 20 (4.2 liters of retentate). Flow rates are shown in Table 2.

After the bGH had been concentrated, the solution was desalted by dialysis against NH₄OH using the Pellicon® System with the 10K M.W. cutoff cassette (diafiltration). 3 mM NH₄OH solution (25 volumes per volume of retentate or 105 liters 3 mM NH₄OH in this case) was continuously added to the retentate at a rate just sufficient to make up for the volume loss by diafiltration. The diafiltrate was then discarded. It contains salts and 10% of the input bGH as measured by absorbance. The retentate containing the desalted bGH is drained from the Pellicon® Sytem, and the ultrafilter was washed with a small amount of 3 mM NH₄OH which is combined with the retentate. The retentate volume before addition of the wash was 4.2 liters (39,000 OD) at a concentration of 14.6 g bGH/L. Flow rates are shown in Table 3.

**Table 2**

| Flow Rates for 10K M.W. Ultrafiltration¹ | | |
|---|---|---|
| Conditions | Rate per unit of membrane area | Rate with 3 cassettes (15 ft²) |
| Ultrafiltration rate when retentate is most concentrated: | 1.8 L/hr ft² | 27 L/hr |
| Ultrafiltration rate when retentate is most dilute: | 2.4 L/hr ft² | 36 L/hr |
| Recirculation rate when retentate is most concentrated: | 9 L/hr ft² | 2.25 L/min |
| Recirculation rate when retentate is most dilute: | 6 L/hr ft² | 1.5 L/min |

| | | |
|---|---|---|
| ¹ At an inlet pressure of 20 psig and a back pressure of 10 psig. | | |

**Table 3**

| Flow Rates for Diafiltration¹ against 3 mM NH₄OH | | |
|---|---|---|
| Conditions | Rate per unit of membrane area | Rate with 3 cassettes (15 ft²) |
| Ultrafiltration rate | 2.4 L/hr ft² | 36 L/hr |
| Recirculation rate | 9 L/hr ft² | 2.25 L/min |

| | | |
|---|---|---|
| ¹ At an inlet pressure of 15 psig and a back pressure of 5 psig. | | |

Assay method: Absorbance at 280 nm (1-cell)
Step yield: 90 % Overall yield: 25%
Output Volume: 0.84% of initial cell suspension volume
Calculated assay: 14.6 g bGH/L
Enrichment factor: 18.3

### EXAMPLE 13

### Freeze Drying

The retentate and wash from the dialysis step (4.2 liters, 39,000 OD at a concentration of 14.6 g bGH/L) was shell frozen in a tall glass jar. The jar was connected to a lab freeze dryer for 24 to 48 hours until the contents were lyophilized to dryness. The outer surface of the jar was exposed to ambient temperature throughout the drying cycle. There was no detectable loss of bGH. The bGH (61.3 g) was harvested as a fluffy, white powder.
Assay method: Absorbance on input and release assays on dry bGh
Step yield: 100% Overall yield: 25%
Output volume: 0.012% of initial cell suspension volume
Calculated assay: 100% mash volume
Enrichment factor: 68

## Claims

1. A method for recovering a purified animal or human growth hormone or a polypeptide analog thereof from a bacterial cell in which the eucaryotic growth hormone or analog has been produced by means of expression of a DNA sequence encoding the hormone or analog which comprises the following steps:
a) disrupting the cell wall of the bacterial cell or fragments thereof to produce a lysate;
b) separating a precipitate containing particulate and insoluble material from the lysate;
c) preparing a suspension of the separated precipitate in an appropriate buffer solution;
d) contacting the suspension for a suitable period of time with up to 100 µg lysozyme/ml solution in the absence of DNAse and treating the suspension to effect contact between liquid and solids;
e) separating partially or completely precipitated material from the liquid solids contacted suspension;
f) solubilizing the separated precipitate in a sufficient amount of aqueous solution and adjusting the pH to between 9.0 and 12.0 to form a solution;
g) separating the solubilized hormone or analog from other cellular components by ultrafiltration using a membrane having a cut-off point of about 100 K molecular weight under suitable conditions to produce an ultrafiltrate containing the hormone or analog and a retentate; and
h) treating the solubilized hormone or analog so as to further purify and concentrate the hormone or analog and thereby recover the purified hormone or analog.

2. A method of claim 1, wherein the hormone or analog is an animal growth hormone or an analog thereof.

3. A method of claim 2, wherein the hormone or analog is bovine growth hormone or an analog thereof.

4. A method of claim 3, wherein the analog comprises the amino acid sequence Met-Asp-Gln added to the N-terminus of the phenylalanine form of natural bovine growth hormone and the bacterial cell is Escherichia coli strain ATCC No. 39806.

5. A method of claim 3, wherein the analog comprises a methionine residue added to the N-terminus of the phenylalanine form of natural bovine growth hormone and the bacterial cell is Escherichia coli strain ATCC No. 39785.

6. A method of claim 1, wherein the hormone or analog is human growth hormone or an analog thereof.

7. A method of claim 6, wherein the analog comprises the amino acid sequence of natural human growth hormone beginning at Met¹⁴ and the bacterial cell is Escherichia coli strain ATCC No. 39386.

8. A method of claim 6, wherein the analog comprises the amino acid sequence of natural human growth hormone preceded by methionine at the N-terminus and the bacterial cell is Escherichia coli strain ATCC No. 39384.

9. A method of claim 2, wherein the hormone is porcine growth hormone or an analog thereof.

10. A method of claim 9, wherein the analog comprises a methionine residue added to the N-terminus of the phenylalanine form of natural porcine growth hormone and the bacterial cell is Escherichia coli strain ATCC No. 39804.

11. A method of claim 2, wherein the hormone or analog is chicken growth hormone or an analog thereof.

12. A method of claim 11, wherein the analog comprises a methionine residue added to the N-terminus of the phenylalanine form of natural chicken growth hormone and the bacterial cell is Escherichia coli strain ATCC No. 39792.

13. A method of claim 1, wherein prior to step (a) the bacterial cell or fragments thereof is maintained in suspension in the fermentation mash or is suspended in a suitable buffered solution at a neutral pH.

14. A method of claim 13, wherein the neutral pH is about 7.4.

15. A method of claim 1, wherein in step (a) the cells are mechanically disrupted.

16. A method of claim 1, wherein the lysate is diluted with a suitable amount of deionized water before the particulate material is separated.

17. A method of claim 1, wherein the separation in step (b) or step (e) comprises centrifugation.

18. A method of claim 1, wherein the separation in step (b) or step (e) comprises ultrafiltration.

19. A method of claim 1, wherein the treatment in step (d) to effect contact between liquid and solids comprises sonication.

20. A method of claim 1 which further comprises adding to the suspension a suitable amount of an appropriate surfactant and further incubating the suspension for a suitable period of time prior to effecting liquid-solids contact of the suspension in step (d).

21. A method of claim 1, wherein the precipitated material separated partially or completely from the liquid-solids contacted suspension in step (e) is re-suspended in a suitable amount of an appropriate medium, the suspension so prepared is liquid-solids contacted under suitable conditions, and the resultant precipitated material is separated from the liquid-solids contacted suspension prior to solubilizing in step (f).

22. A method of claim 21, wherein the appropriate medium is a buffered or unbuffered aqueous solution.

23. A method of claim 21, wherein the resultant precipitate is re-suspended, liquid-solids contacted and separated at least one additional time.

24. A method of claim 1, wherein after solubilizing the precipitate at a pH of about 9.0-12.0, the pH is adjusted to about 10.5

25. A method of claim 1, wherein an appropriate amount of a suitably concentrated aqueous solution of sodium borate is added to the solution obtained in step (f).

26. A method of claim 1 which further comprises clarifying the solution obtained in step (f) by liquid-solids contacting of the solution under appropriate conditions, centrifuging the liquid-solids contacted solution under suitable conditions, separating the supernatant liquid and filtering the liquid to produce a clarified solution containing the hormone or analog.

27. A method of claim 1, wherein the retentate obtained in step (g) is resubjected to ultrafiltration at least once prior or subsequent to the treatment in step (h).

28. A method of claim 27, wherein the retentate is diluted with a suitable buffer at an appropriate alkaline pH before being re-subjected to ultrafiltration.

29. A method of claim 28, wherein the appropriate alkaline pH is about 9.0-12.0.

30. A method of claim 28, wherein the dilution and ultrafiltration are repeated at least once.

31. A method of claim 27, wherein the retentate is maintained at a substantially constant volume during ultrafiltration.

32. A method of claim 30, wherein the ultrafiltration at substantially constant retentate volume is repeated at least once.

33. A method of claim 1, wherein the treatment of the solubilized hormone in step (h) comprises ion exchange chromatography.

34. A method of claim 32, wherein the ion exchange chromatography is effected with an amino-containing ion exchange resin.

35. A method of claim 33, wherein the amine-containing ion exchange resin is diethylaminoethyl-dextran or -sepharose.

36. A method of claim 1, wherein the purified hormone or analog is concentrated by ultrafiltration.

37. A method of claim 1, wherein the purified hormone or analog is further purified by dialysis followed by drying.

38. A method of claim 36, wherein the dialysis is against a solution containing a suitable concentration of an appropriate salt.

39. A method of claim 37, wherein the appropriate salt is ammonium hydroxide or ammonium bicarbonate.

40. A method of claim 36, wherein the drying is effected by lyophilization.

41. A method of claim 36, wherein the drying is effected by spray drying under appropriate conditions.

## Patentansprüche

1. Verfahren zur Gewinnung eines gereinigten tierischen oder menschlichen Wachstumshormons oder eines Polypeptid-Analogen desselben aus einer Bakterienzelle, in der das eucaryontische Wachstumshormon oder dessen Analoges durch Expression einer DNA-Sequenz mit Codierung für das Hormon oder dessen Analoges gebildet wurde, umfassend folgende Stufen:
a) Aufbrechen der Zellwand der Bakterienzelle oder von Fragmenten derselben zur Bildung eines Lysats;
b) Abtrennen eines teilchenförmiges und unlösliches Material enthaltendenden Niederschlags aus dem Lysat;
c) Zubereiten einer Suspension des abgetrennten Niederschlags in einer geeigneten Pufferlösung;
d) In-Berührung-Bringen der Suspension während einer geeigneten Zeitdauer mit einer Lösung mit bis zu 100 µg Lysozym/ml in Abwesenheit von DNAse und Behandeln der Suspension zur Herbeiführung eines Kontakts zwischen Flüssigkeit und Feststoffen;
e) teilweises oder vollständiges Abtrennen von ausgefälltem Material aus der Suspension, in der die Flüssigkeit mit den Feststoffen in Kontakt gebracht wurde;
f) In-Lösung-Bringen des abgetrennten Niederschlags in einer ausreichenden Menge einer wäßrigen Lösung und Einstellen des pH-Werts zwischen 9,0 und 12,0 zur Bildung einer Lösung;
g) Abtrennen des in Lösung gebrachten Hormons oder Analogen von den sonstigen Zellkomponenten durch Ultrafiltration unter Verwendung einer Membran eines Abtrennungspunkts von etwa 100 K Molekulargewicht unter geeigneten Bedingungen zur Gewinnung eines das Hormon oder Analoge und ein Retentat enthaltenden Ultrafiltrats und
h) Behandeln des in Lösung gebrachten Hormons oder Analogen zur weiteren Reinigung und Konzentration des Hormons oder Analogen unter Gewinnung des gereinigten Hormons oder Analogen.

2. Verfahren nach Anspruch 1, wobei das Hormon oder Analoge aus einem tierischen Wachstumshormon oder einem Analogen desselben besteht.

3. Verfahren nach Anspruch 2, wobei das Hormon oder Analoge aus Rinderwachstumshormon oder einem Analogen desselben besteht.

4. Verfahren nach Anspruch 3, wobei das Analoge die an das N-Ende der Phenylalanin-Form natürlichen Rinderwachstumshormons addierte Aminosäuresequenz Met-Asp-Gln aufweist und die Bakterienzelle aus dem Escherichia coli-Stamm ATCC Nr. 39806 besteht.

5. Verfahren nach Anspruch 3, wobei das Analoge einen an das N-Ende der Phenylalanin-Form natürlichen Rinderwachstumshormons addierten Methionin-Rest aufweist und die Bakterienzelle aus dem Escherichia coli-Stamm ATCC Nr. 39785 besteht.

6. Verfahren nach Anspruch 1, wobei das Hormon oder Analoge aus menschlichem Wachstumshormon oder einem Analogen desselben besteht.

7. Verfahren nach Anspruch 6, wobei das Analoge die Aminosäure-Sequenz natürlichen menschlichen Wachstumshormons, beginnend bei Met¹⁴ , aufweist und die Bakterrienzelle aus dem Escherichia coli-Stamm ATCC Nr. 39386 besteht.

8. Verfahren nach Anspruch 6, wobei das Analoge die Aminosäure-Sequenz von natürlichem menschlichen Wachstumshormon mit dem N-Ende vorangehendem Methionin aufweist und die Bakterienzelle aus dem Escherichia coli-Stamm ATCC Nr. 39384 besteht.

9. Verfahren nach Anspruch 2, wobei das Hormon aus Schweinewachstumshormon oder einem Analogen desselben besteht.

10. Verfahren nach Anspruch 9, wobei das Analoge einen am N-Ende der Phenylalanin-Form natürlichen Schweinewachstumshormons addierten Methionin-Rest aufweist und die Bakterienzelle aus dem Escherichia coli-Stamm ATCC Nr. 39804 besteht.

11. Verfahren nach Anspruch 2, wobei das Hormon oder Analoge aus Geflügelwachstumshormon oder einem Analogen desselben besteht.

12. Verfahren nach Anspruch 11, wobei das Analoge einen am N-Ende der Phenylalanin-Form natürlichen Geflügelwachstumshormons addierten Methionin-Rest aufweist und die Bakterienzelle aus dem Escherichia coli-Stamm ATCC Nr. 39792 besteht.

13. Verfahren nach Anspruch 1, wobei vor der Stufe (a) die Bakterienzelle oder Fragmente derselben in der Fermentationsmaische suspendiert gehalten oder in einer geeigneten Pufferlösung bei einem neutralen pH-Wert suspendiert wird (werden).

14. Verfahren nach Anspruch 13, wobei der neutrale pH-Wert etwa 7,4 beträgt.

15. Verfahren nach Anspruch 1, wobei in Stufe (a) die Zellen mechanisch aufgebrochen werden.

16. Verfahren nach Anspruch 1, wobei das Lysat vor Abtrennung des teilchenförmigen Materials mit einer geeigneten Menge entionisierten Wassers verdünnt wird.

17. Verfahren nach Anspruch 1, wobei die Abtrennung in Stufe (b) oder Stufe (e) ein Zentrifugieren umfaßt.

18. Verfahren nach Anspruch 1, wobei die Abtrennung in Stufe (b) oder Stufe (e) eine Ultrafiltration umfaßt.

19. Verfahren nach Anspruch 1, wobei die Behandlung in Stufe (d) zur Herbeiführung eines Kontakts zwischen Flüssigkeit und Feststoff eine Beschallung umfaßt.

20. Verfahren nach Anspruch 1, bei welchem des weiteren der Suspension eine geeignete Menge eines geeigneten oberflächenaktiven Mittels zugesetzt und ferner die Suspension vor Herbeiführung des Kontakts zwischen Flüssigkeit und Feststoffen in der Suspension in Stufe (d) eine geeignete Zeit lang inkubiert wird.

21. Verfahren nach Anspruch 1, wobei das teilweise oder vollständig aus der Suspension, in der Flüssigkeit und Feststoffe in Kontakt gebracht worden sind, in Stufe (e) abgetrennte ausgefällte Material in einer geeigneten Menge eines geeigneten Mediums resuspendiert wird, die derart zubereitete Suspension unter geeigneten Bedingungen einen Flüssigkeits-/Feststoff-Kontakt erfährt und das gebildete ausgefällte Material von der Suspension, in der der Flüssigkeits-/Feststoff-Kontakt stattgefunden hat, vor dem In-Lösung-Bringen in Stufe (f) abgetrennt wird.

22. Verfahren nach Anspruch 21, wobei das geeignete Medium aus einer gepufferten oder ungepufferten wäßrigen Lösung besteht.

23. Verfahren nach Anspruch 21, wobei der gebildete Niederschlag mindestens ein weiteres Mal resuspendiert, einem Flüssigkeits-/Feststoff-Kontakt unterworfen und abgetrennt wird.

24. Verfahren nach Anspruch 1, wobei nach dem In-Lösung-Bringen des Niederschlags bei einem pH-Wert von etwa 9,0 - 12,0 der pH-Wert auf etwa 10,5 eingestellt wird.

25. Verfahren nach Anspruch 1, wobei eine geeignete Menge einer in geeigneter Weise konzentrierten wäßrigen Natriumborat-Lösung der in Stufe (f) erhaltenen Lösung zugesetzt wird.

26. Verfahren nach Anspruch 1, bei welchem des weiteren die in Stufe (f) erhaltene Lösung durch Flüssigkeits-/Feststoff-Kontaktieren der Lösung unter geeigneten Bedingungen geklärt, die einem Flüssigkeits-/Feststoff-Kontakt unterworfene Lösung unter geeigneten Bedingungen zentrifugiert, der flüssige Überstand abgetrennt und die Flüssigkeit filtriert wird, um eine das Hormon oder das Analoge enthaltende geklärte Lösung herzustellen.

27. Verfahren nach Anspruch 1, wobei das in Stufe (g) erhaltene Retentat vor oder nach der Behandlung in Stufe (h) noch mindestens einmal ultrafiltriert wird.

28. Verfahren nach Anspruch 27, wobei das Retentat vor der erneuten Ultrafiltration mit einem geeigneten Puffermittel auf einen geeigneten alkalischen pH-Wert verdünnt wird.

29. Verfahren nach Anspruch 28, wobei der geeignete alkalische pH-Wert etwa 9,0 - 12,0 beträgt.

30. Verfahren nach Anspruch 28, wobei das Verdünnen und Ultrafiltrieren mindestens einmal wiederholt werden.

31. Verfahren nach Anspruch 27, wobei das Retentat während des Ultrafiltrierens auf praktisch konstantem Volumen gehalten wird.

32. Verfahren nach Anspruch 31, wobei das Ultrafiltrieren bei praktischem konstantem Retentat-Volumen mindestens einmal wiederholt wird.

33. Verfahren nach Anspruch 1, wobei die Behandlung des in Lösung gebrachten Hormons in Stufe (h) eine Ionenaustausch-Chromatographie umfaßt.

34. Verfahren nach Anspruch 33, wobei die Ionenaustausch-Chromatographie mit einem aminhaltigen Ionenaustauscherharz durchgeführt wird.

35. Verfahren nach Anspruch 34, wobei das aminhaltige Ionenaustauscherharz aus Diethylaminoethyldextran oder -sepharose besteht.

36. Verfahren nach Anspruch 1, wobei das gereinigte Hormon oder das Analoge durch Ultrafiltrieren konzentriert wird.

37. Verfahren nach Anspruch 1, wobei das gereinigte Hormon oder das Analoge im Anschluß an das Trocknen durch Dialyse weiter gereinigt wird.

38. Verfahren nach Anspruch 37, wobei gegen eine Lösung mit einer geeigneten Konzentration eines geeigneten Salzes dialysiert wird.

39. Verfahren nach Anspruch 38, wobei das geeignete Salz aus Ammoniumhydroxid oder Ammoniumbicarbonat besteht.

40. Verfahren nach Anspruch 37, wobei durch Lyophilisieren getrocknet wird.

41. Verfahren nach Anspruch 37, wobei durch Sprühtrocknen unter geeigneten Bedingungen getrocknet wird.

## Revendications

1. Procédé de récupération d'une hormone de croissance animale ou humaine purifiée ou d'un polypeptide analogue de celle-ci, à partir d'une cellule bactérienne, selon lequel l'hormone de croissance eucaryote ou l'analogue, a été produit par expression d'une séquence d'ADN codant pour l'hormone ou l'analogue, comprenant les étapes consistant :
a) à désintégrer la paroi cellulaire de la cellule bactérienne ou des fragments de celle-ci, pour produire un lysat ;
b) à séparer un précipité contenant une matière particulaire et insoluble, à partir du lysat ;
c) à préparer une suspension du précipité séparé, dans une solution tampon appropriée ;
d) à mettre en contact la suspension pendant une période appropriée, avec jusqu'à 100 µg de lysozyme/ml de solution, en l'absence d'ADNase, et à traiter la suspension pour mettre en contact le liquide et les solides ;
e) à séparer partiellement ou complètement la matière précipitée à partir de la suspension de liquide et de solides mis en contact ;
f) à dissoudre le précipité séparé, dans une quantité suffisante d'une solution aqueuse, et à ajuster le pH à 9,0 jusqu'à 12,0, pour former une solution ;
g) à séparer l'hormone ou l'analogue dissous, à partir d'autres composants cellulaires, par ultrafiltration en employant une membrane ayant un point de fractionnement à un poids moléculaire d'environ 100 K dans des conditions appropriées, pour produire un ultrafiltrat contenant l'hormone ou un analogue, et une fraction retenue ; et
h) à traiter l'hormone ou l'analogue dissous, de façon à purifier et concentrer encore l'hormone ou l'analogue, et récupérer ainsi l'hormone ou l'analogue purifié.

2. Procédé selon la revendication 1, dans lequel l'hormone ou l'analogue, est une hormone de croissance animale ou un analogue de celle-ci.

3. Procédé selon la revendication 2, dans lequel l'hormone ou l'analogue, est une hormone de croissance bovine ou un analogue de celle-ci.

4. Procédé selon la revendication 3, dans lequel l'analogue comprend la séquence d'aminoacides Met-Asp-Gln ajoutée à l'extrémité N-terminale de la forme à résidu phénylalanine de l'hormone de croissance bovine naturelle, et dans lequel la cellule bactérienne est Escherichia coli de la souche ATCC N° 39806.

5. Procédé selon la revendication 3, dans lequel l'analogue comprend un résidu méthionine ajouté à l'extrémité N-terminale de la forme à résidu phénylalanine de l'hormone de croissance bovine naturelle, et dans lequel la cellule bactérienne est Escherichia coli de la souche ATCC N° 39785.

6. Procédé selon la revendication 1, dans lequel l'hormone ou l'analogue, est une hormone de croissance humaine ou un analogue de celle-ci.

7. Procédé selon la revendication 6, dans lequel l'analogue comprend la séquence d'aminoacides de l'hormone de croissance humaine naturelle, commençant au résidu Met¹⁴, et dans lequel la cellule bactérienne est Escherichia coli de la souche ATCC N° 39386.

8. Procédé selon la revendication 6, dans lequel l'analogue comprend la séquence d'aminoacides de l'hormone de croissance humaine naturelle précédée par une méthionine à l'extrémité N-terminale, et dans lequel la cellule bactérienne est Escherichia coli de la souche ATCC N° 39384.

9. Procédé selon la revendication 2, dans lequel l'hormone est l'hormone de croissance porcine ou un analogue de celle-ci.

10. Procédé selon la revendication 9, dans lequel l'analogue comprend un résidu méthionine ajouté à l'extrémité N-terminale de la forme à résidu phénylalanine de l'hormone de croissance porcine naturelle, et dans lequel la cellule bactérienne est Escherichia coli de la souche ATCC N° 39804.

11. Procédé selon la revendication 2, dans lequel l'hormone ou l'analogue, est l'hormone de croissance de poulet ou un analogue de celle-ci.

12. Procédé selon la revendication 11, dans lequel l'analogue comprend un résidu méthionine ajouté à l'extrémité N-terminale de la forme à résidu phénylalanine de l'hormone de croissance de poulet naturelle, et dans lequel la cellule bactérienne est Escherichia coli de la souche ATCC N° 39792.

13. Procédé selon la revendication 1, dans lequel avant l'étape (a), la cellule bactérienne ou des fragments de celle-ci, sont maintenus en suspension dans le milieu de fermentation, ou ils sont mis en suspension dans une solution tamponnée de manière appropriée à un pH neutre.

14. Procédé selon la revendication 13, dans lequel le pH neutre est d'environ 7,4.

15. Procédé selon la revendication 1, dans lequel dans l'étape (a), les cellules sont désintégrées de manière mécanique.

16. Procédé selon la revendication 1, dans lequel le lysat est dilué avec une quantité appropriée d'eau désionisée avant de séparer la matière particulaire.

17. Procédé selon la revendication 1, dans lequel la séparation effectuée dans l'étape (b) ou l'étape (e), comprend une centrifugation.

18. Procédé selon la revendication 1, dans lequel la séparation effectuée dans l'étape (b) ou (e), comprend une ultrafiltration.

19. Procédé selon la revendication 1, dans lequel le traitement effectué dans l'étape (d) pour mettre en contact le liquide et les solides, comprend une sonication.

20. Procédé selon la revendication 1, comprenant en outre l'addition dans la suspension, d'une quantité appropriée d'un tensioactif approprié, et en outre l'incubation de la suspension pendant une période appropriée avant d'effectuer le contact liquide-solides de la suspension dans l'étape (d).

21. Procédé selon la revendication 1, dans lequel la matière précipitée séparée partiellement ou complètement à partir de la suspension de liquide et de solides mis en contact dans l'étape (e), est remise en suspension dans une quantité appropriée d'un milieu adapté, la suspension ainsi préparée est constituée de liquide et de solides mis en contact dans des conditions appropriées, et la matière précipitée résultante est séparée de la suspension de liquide et de solides mis en contact, avant la dissolution effectuée dans l'étape (f).

22. Procédé selon la revendication 21, dans lequel le milieu approprié est une solution aqueuse tamponnée ou non tamponnée.

23. Procédé selon la revendication 21, dans lequel le précipité résultant est remis en suspension, et le liquide et les solides sont mis en contact et séparés après au moins une période supplémentaire.

24. Procédé selon la revendication 1, dans lequel après dissolution du précipité à un pH d'environ 9,0 à 12,0, le pH est ajusté à environ 10,5.

25. Procédé selon la revendication 1, dans lequel on ajoute dans la suspension obtenue dans l'étape (f), une quantité appropriée d'une solution aqueuse de borate de sodium concentrée de manière appropriée.

26. Procédé selon la revendication 1, comprenant en outre la clarification de la solution obtenue dans l'étape (f), en mettant en contact le liquide et les solides de la solution dans des conditions appropriées, par centrifugation de la solution à liquide et solides mis en contact, dans des conditions appropriées, en séparant le liquide surnageant et en filtrant le liquide pour produire une solution clarifiée contenant l'hormone ou l'analogue.

27. Procédé selon la revendication 1, dans lequel la fraction retenue, obtenue dans l'étape (g) est resoumise à une ultrafiltration, au moins une fois, avant ou après le traitement effectué dans l'étape (h).

28. Procédé selon la revendication 27, dans lequel la fraction retenue, est diluée avec un tampon approprié à un pH alcalin approprié, avant d'être resoumise à une ultrafiltration.

29. Procédé selon la revendication 28, dans lequel le pH alcalin approprié, est d'environ 9,0 à 12,0.

30. Procédé selon la revendication 28, dans lequel la dilution et l'ultrafiltration sont répétées au moins une fois.

31. Procédé selon la revendication 27, dans lequel la fraction retenue est maintenue à un volume à peu près constant au cours de l'ultrafiltration.

32. Procédé selon la revendication 31, dans lequel l'ultrafiltration à volume de fraction retenue à peu près constant, est répétée au moins une fois.

33. Procédé selon la revendication 1, dans lequel le traitement de l'hormone dissoute effectué dans l'étape (h), comprend une chromatographie par échange d'ions.

34. Procédé selon la revendication 33, dans lequel la chromatographie par échange d'ions, est effectuée avec une résine échangeuse d'ions aminée.

35. Procédé selon la revendication 34, dans lequel la résine échangeuse d'ions aminée, est du diéthylaminoéthyl-dextrane ou sépharose.

36. Procédé selon la revendication 1, dans lequel l'hormone ou l'analogue purifié, est concentré par ultrafiltration.

37. Procédé selon la revendication 1, dans lequel l'hormone ou l'analogue purifié, est en outre purifié par dialyse suivie par un séchage.

38. Procédé selon la revendication 37, dans lequel la dialyse est effectuée contre une solution ayant une concentration appropriée en un sel approprié.

39. Procédé selon la revendication 38, dans lequel le sel approprié, est l'hydroxyde d'ammonium ou le bicarbonate d'ammonium.

40. Procédé selon la revendication 37, dans lequel le séchage est effectué par lyophilisation.

41. Procédé selon la revendication 37, dans lequel le séchage est effectué par séchage par atomisation dans des conditions appropriées.
